(19) Europäisches Patentamt
European Patent Office
Office européen des brevets

(11) EP 0 875 240 A2

# (12) EUROPÄISCHE PATENTANMELDUNG

(43) Veröffentlichungstag:
**04.11.1998 Patentblatt 1998/45**

(51) Int. Cl.$^6$: **A61K 7/42**

(21) Anmeldenummer: **98106686.3**

(22) Anmeldetag: **11.04.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **30.04.1997 DE 19718318**

(71) Anmelder:
**Beiersdorf Aktiengesellschaft**
**20245 Hamburg (DE)**

(72) Erfinder:
- **Scheel, Oliver, Dr.**
  **40489 Düsseldorf (DE)**
- **Gers-Barlag, Heinrich, Dr.**
  **25495 Kummerfeld (DE)**
- **Kröpke, Rainer**
  **22527 Hamburg (DE)**

## (54) Wirkstoffkombinationen bzw. Addukte aus Dibenzoylmethanderivaten und/oder Zimtsäurederivaten und Cyclodextrinen und Verwendung solcher Wirkstoffkombinationen in kosmetischen Lichtschutzzubereitungen

(57) Wirkstoffkombinationen, insbesondere molekulare Addukte, aus Cyclodextrinen und kosmetisch oder dermatologisch unbedenklichen Substanzen, deren chemische Formel das Strukturmotiv des Dibenzoylmethans und/oder der Zimtsäure enthalten.

EP 0 875 240 A2

## Beschreibung

Die vorliegende Erfindung betrifft neue lichtschutzaktive, photostabile Wirkstoffkombinationen bzw. in Form molekularer Addukte vorliegende lichtschutzaktive, photostabile Wirkstoffkombinationen für kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Eine bekannte und vorteilhafte Lichtschutzfiltersubstanz ist das 4-(tert.-Butyl)-4'-methoxydibenzoylmethan, welches sich durch die Struktur

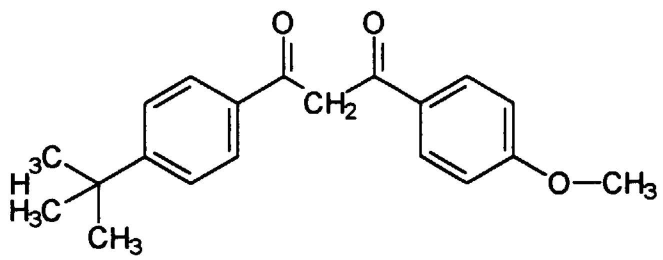

auszeichnet, und von Givaudan unter der Marke Parsol® 1789 verkauft wird

Der Hauptnachteil dieser Substanz ist eine gewisse Instabilität gegenüber UV-Strahlung, so daß es zweckmäßig ist, Zubereitungen mit einem Gehalt an dieser Substanz auch gewisse UV-Stabilisatoren einzuverleiben. Die photochemische Zersetzung von 4-(tert.-Butyl)-4'-methoxydibenzoylmethan - stellvertretend für alle im UV-Bereich absorbierenden Dibenzoylmethanderivate - folgt einer Norrish-Typ-I-Acylspaltung gemäß dem nachfolgenden Reaktionsschema:

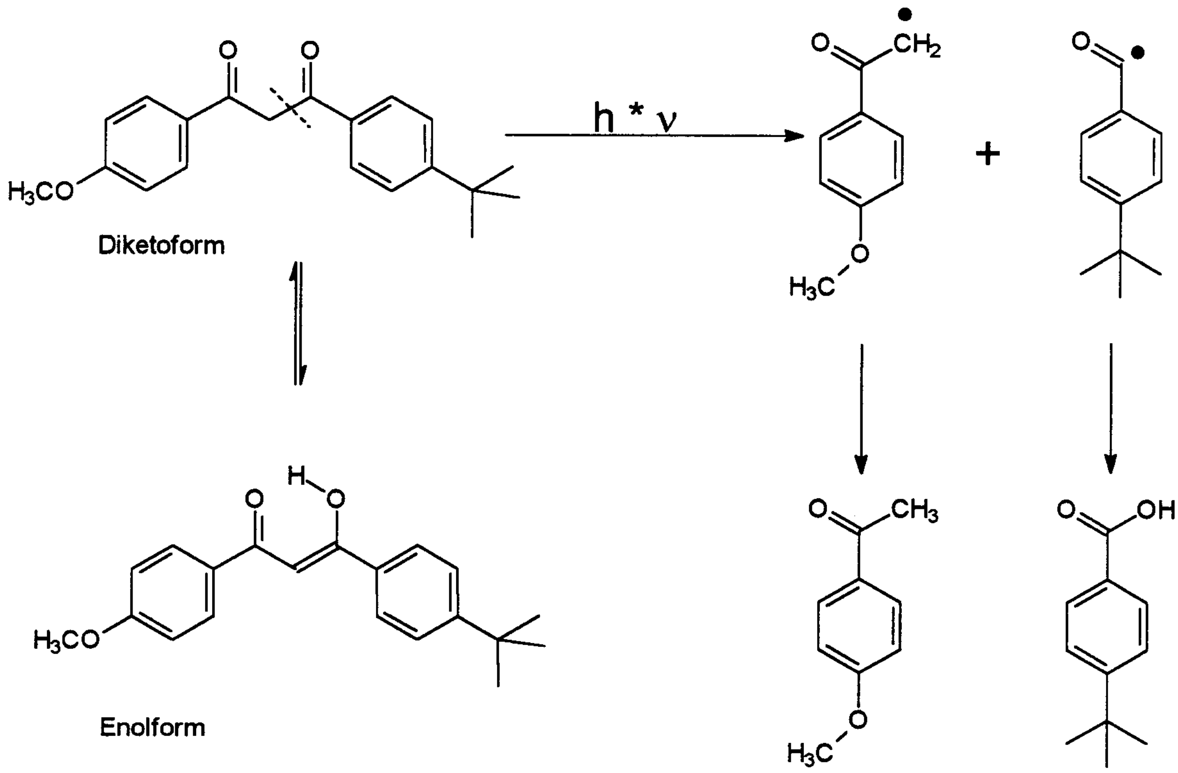


Diketoform

Enolform

Die Reaktionsprodukte stehen als Lichtschutzfiltersubstanzen nicht mehr zur Verfügung. Es war daher ein dringender Bedarf Wege aufzuweisen, auf welchen der photolytischen Zersetzung von Dibenzoylmethanderivaten wirksam begegnet werden kann.

Beispielsweise beschreibt die deutsche Offenlegungsschrift DE-A-37 41 420 die Kombination dieses Lichtschutzfilters in bestimmtem Mengenverhältnis zu 4-Methylbenzylidencampher, welcher sich durch die Struktur

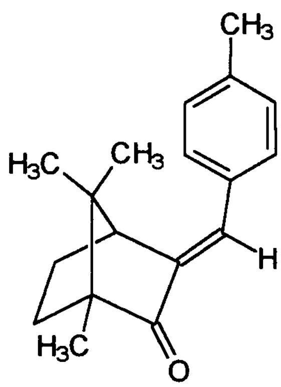

auszeichnet und von Merck unter der Marke Eusolex® 6300 verkauft wird. Die a.a.O. beschriebenen Zubereitungen zeichnen sich aber wiederum durch andere Nachteile aus, hauptsächlich formulierungstechnischer Natur.

Eine weiterere vorteilhafte Lichtschutzfiltersubstanz ist das 2-Ethylhexyl-p-methoxy-cinnamat (4-Methxoyzimtsäure-2'ethylhexylhester), welches von Givaudan unter der Bezeichnung Parsol® MCX erhältlich ist und sich durch folgende Struktur auszeichnet:

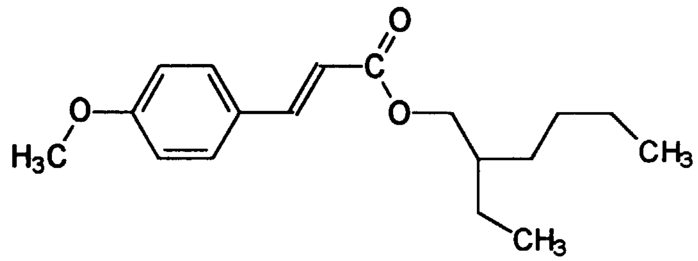

Der Hauptnachteil auch dieser Substanz ist eine gewisse Instabilität gegenüber UV-Strahlung.

Für Gemische aus Cinnamaten und Dibenzoylmethanen gilt in der Regel Gleiches. Im Gegensatz zu manchen Lichtschutzfilterkombinationen, die sich durch erhöhte Stabilität gegenüber den jeweiligen Einzelsubstanzen auszeichnen, wird unter gewissen Umständen für Gemische aus Cinnamaten und Dibenzoylmethanen sogar eine stärkere Destabilisierung gegenüber UV-Licht beobachtet als für die jeweiligen Einzelsubstanzen. Dies ist insbesondere der Fall bei Gemischen aus 2-Ethylhexyl-p-methoxycinnamat und 4-(tert.-Butyl)-4'-methoxydibenzoylmethan. Mit einiger Sicherheit reagieren diese beiden Komponenten unter UV-Einfluß zu inaktiven Produkten und stehen für die UV-Absorption nicht mehr zur Verfügung.

Den Nachteilen des Standes der Technik abzuhelfen, war also Aufgabe der vorliegenden Erfindung.

Es war indes überraschend und für den Fachmann nicht vorauszusehen, daß Wirkstoffkombinationen aus Cyclodextrinen und kosmetisch oder dermatologisch unbedenklichen Substanzen, deren chemische Formel das Strukturmotiv des Dibenzoylmethans

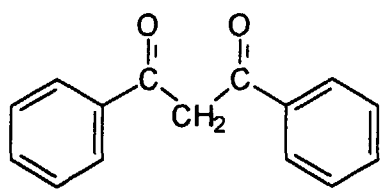

und/oder der Zimtsäure

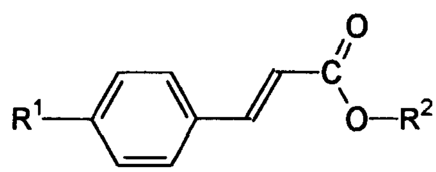

enthalten, den Nachteilen des Standes der Technik abhelfen.

Cyclodextrine (Cycloamylosen, Cycloglucane) sind in kosmetischen und pharmazeutischen Zubereitungen an sich bekannt. Oftmals werden diese Substanzen zur „molekularen Verkapselung" verwendet, also als schützende Umhüllung empfindlicher Moleküle. Es handelt sich dabei um 6, 7, 8 oder noch mehr $\alpha$-1,4-verknüpfte Glucoseeinheiten, wobei die Cyclohexaamylose ($\alpha$-Cyclodextrin) sich durch die Struktur

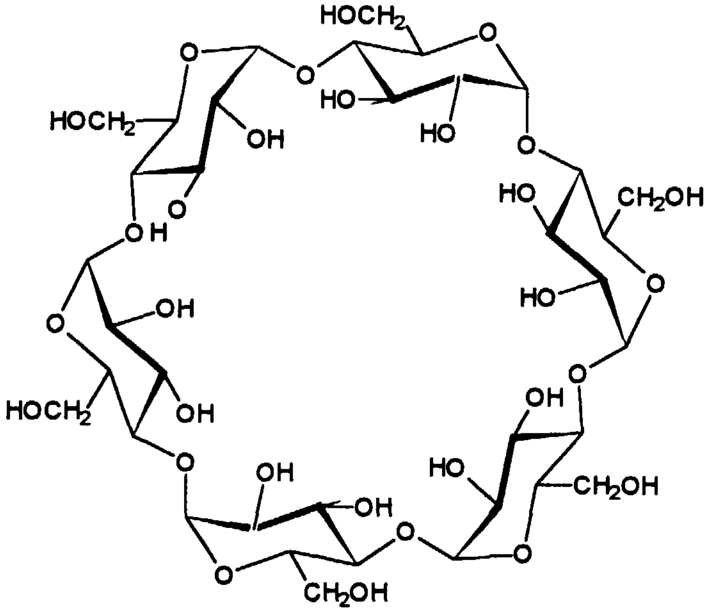

auszeichnet. Die Cycloheptaamylose (β-Cyclodextrin) zeichnet sich durch die Struktur

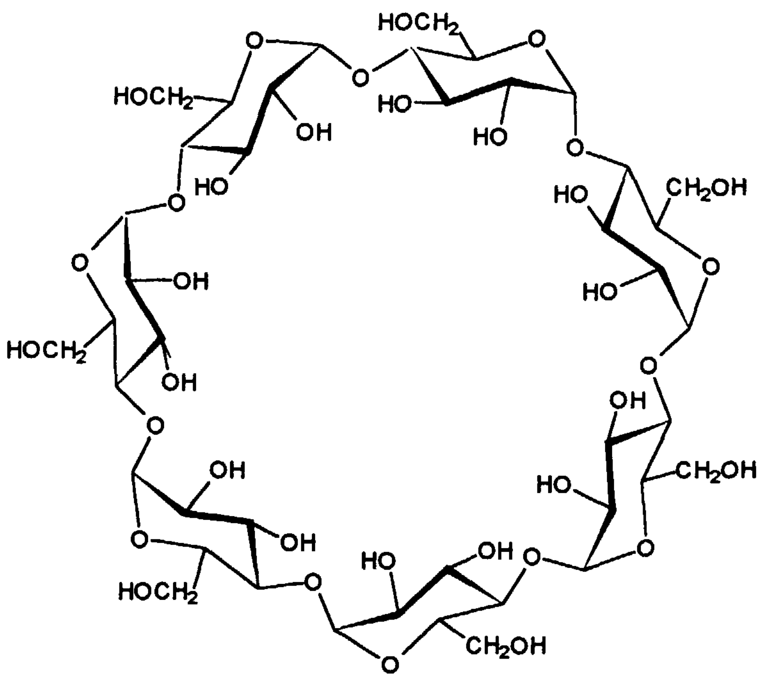

aus. Die Cyclcootaamylose (γ-Cyclodextrin) zeichnet sich durch die Struktur

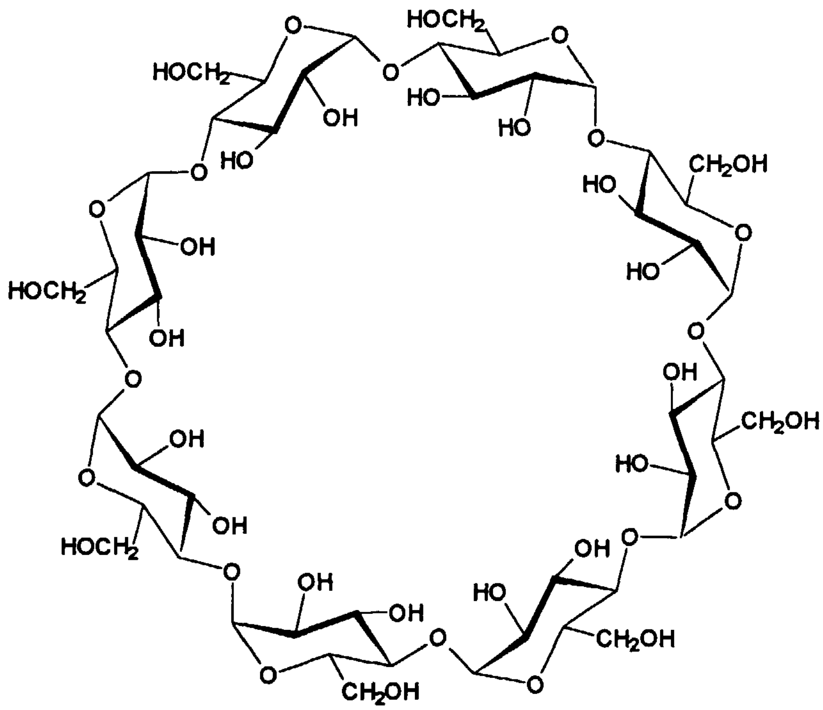

aus. Die Cycloenneaamylose (δ-Cyclodextrin) zeichnet sich durch die Struktur

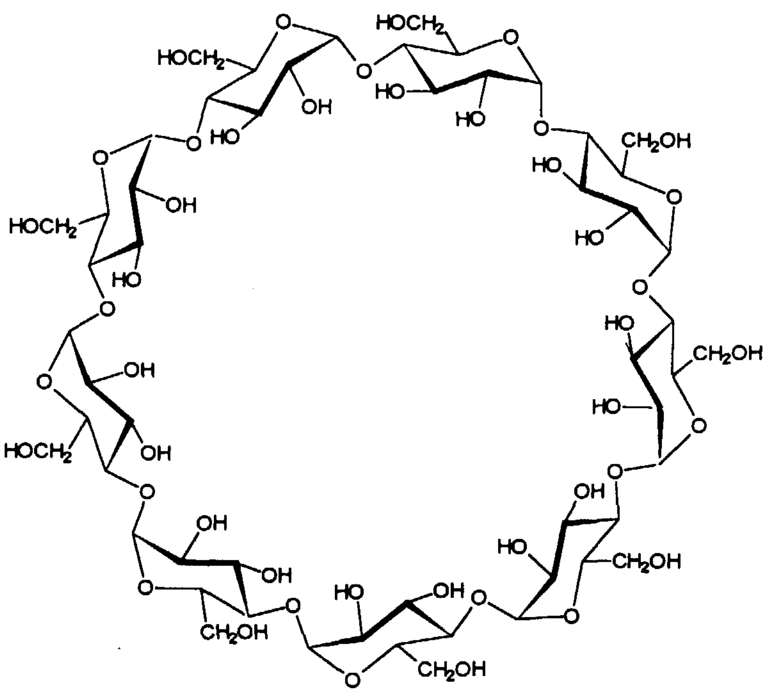

aus.

Es war insbesondere deshalb überraschend für den Fachmann, daß die erfindungsgemäßen Wirkstoffkombinationen photostabil sind, weil die Cyclodextrine sich eben nicht durch nennenswerte Lichtabsorption im UV-Bereich auszeichnen. Sie kamen infolgedessen auch nicht von vorneherein als Stabilisatoren für durch Einwirkung von UV-Licht zersetzliche Substanzen in Frage.

Von den Dibenzoylmethanderivaten werden vorteilhaft verwendet:

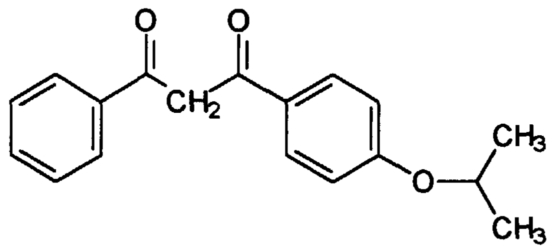

5-Isopropyldibenzoylmethan
CAS-Nr. 63250-25-9

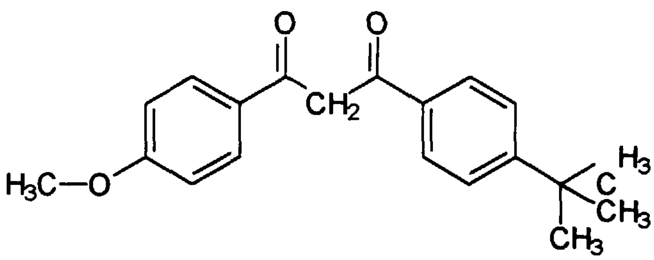

4-(tert.-Butyl)-4'-methoxydibenzoylmethan
CAS-Nr. 70356-09-1

Von den Zimtsäurederivaten werden vorteilhaft verwendet:

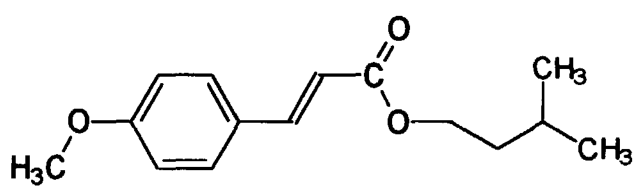

4-Methoxyzimtsäureisopentylester

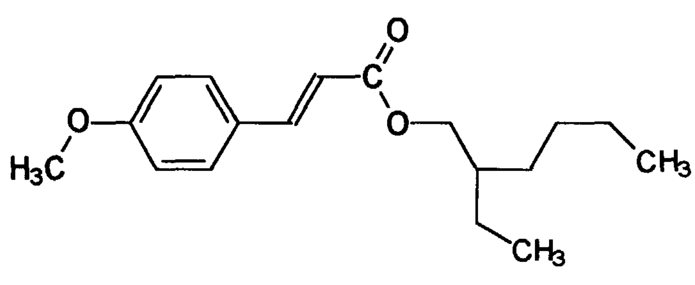

4-Methoxyzimtsäure-2'-ethylhexylester.

Die erfindungsgemäßen Wirkstoffkombinationen lassen sich problemlos üblichen kosmetischen Zubereitungen, vorteilhaft Lichtschutzzubereitungen, aber auch gewünschtenfalls anderen Zubereitungen, beispielsweise pharmazeutischen Zubereitungen einverleiben.

Von großem Vorteil ist, das Cyclodextrin oder die Cyclodextrine einerseits und eine oder mehrere Substanzen, deren chemische Formel das Strukturmotiv des Dibenzoylmethans enthalten andererseits bzw. Cyclodextrin oder die Cyclodextrine einerseits und eine oder mehrere Substanzen, deren chemische Formel das Strukturmotiv der Zimtsäure enthalten, gesondert, also vor der Herstellung der endgültigen kosmetischen oder dermatologischen Zubereitungen,

miteinander zu vereinigen und die vereinigten Produkte dann den Zubereitungen in an sich bekannter Weise zuzufügen.

Es wird vermutet, daß zumindest bei der vorab geschilderten direkten Vereinigung der Substanzen erfindungsgemäß molekulare Addukte aus Cyclodextrinen und Dibenzoylmethanderivaten bzw. molekulare Addukte aus Cyclodextrinen und Zimtsäurederivaten erhalten werden. Es wird daher im folgenden auch von erfindungsgemäßen molekularen Addukten die Rede sein, wenn erfindungsgemäße Wirkstoffkombinationen aus Cyclodextrinen und Dibenzoylmethanderivaten bzw. aus Cyclodextrinen und Zimtsäurederivaten besprochen werden.

Es bestehen gute Gründe, anzunehmen, daß solche molekularen Addukte in Analogie zu anderen molekularen Addukte der Cyclodextrine folgendem Schema folgen:

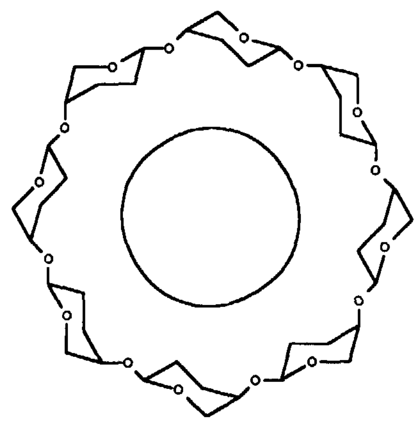

In diesem als wahrscheinlich anzunehmenden Schema stellen die Cyclodextringerüste das Wirtsmolekül und das betreffende Dibenzoylmethanderivat bzw. Zimtsäurederivat, welche hier durch den Kreis im Innern des Schemas dargestellt sind, das Gestmolekül dar.

Aufgrund der errechneten molaren Verhältnisse sind erfindungsgemäß auch Wirkstoffkombinationen erhältlich, welche mit einiger Wahrscheinlichkeit als molekulare Addukte anzusehen sind, in welchen zwei, gegebenenfalls sogar noch mehr, identische oder unterschiedliche Gastmoleküle, welche hier durch Kreise im Innern des Schemas dargestellt sind, in einem Wirtsmolekül gleichsam auf molekularer Ebene verkapselt vorliegen. Dies wird im nachfolgenden Schema angedeutet.

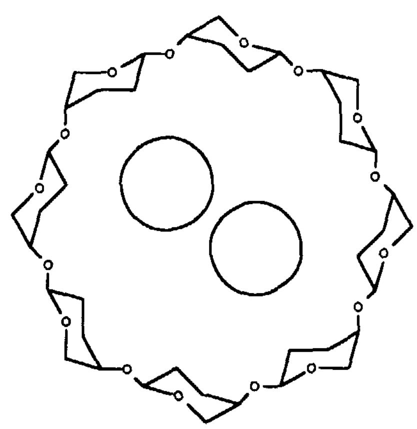

Solche molekularen Addukte bilden sich bevorzugt bei direkter Vereinigung der zugrundeliegenden Einzelsubstanzen, besonders bevorzugt, wenn die Vereinigung unter Vermittlung eines geeigneten Lösemittels erfolgt.

Vorteilhaft können erfindungsgemäße molekulare Addukte aus Cyclodextrinen und Dibenzoylmethanderivaten bzw. molekulare Addukte aus Cyclodextrinen und Zimtsäurederivaten beispielsweise erhalten werden, indem Cylcodextrine in Wasser gelöst werden und das Dibenzoylmethanderivat bzw. das Zimtsäurederivat hinzugegeben werden. Das jeweilige molekulare Addukt fällt sodann als Festkörper aus und kann den üblichen Reinigungs- und Aufbereitungsschritten unterworfen werden.

Bei Befolgen der erfindungsgemäßen Lehre sind Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan und 4-Methoxyzimtsäure-2'-ethylhexylester gegen die Zersetzung unter UV-Licht wird drastisch erhöht.

Ganz besonders erstaunlich war, daß durch Anwendung der erfindungsgemäßen Lehre die Erhöhung der Stabilität von Dibenzoylmethanen und Zimtsäurederivaten, insbesondere von 4-(tert.-Butyl)-4'-methoxydibenzoylmethan und 4-Methoxyzimtsäure-2'-ethylhexylester, sogar dann erfolgt, wenn in ein und derselben kosmetischen Zubereitung Dibenzoylmethane und Zimtsäurederivate gleichzeitig, nämlich in Form der erfindungsgemäßen Wirkstoffkombinationen bzw. der erfindungsgemäßen molekularen Addukte, vorliegen.

Als Verkörperung der vorliegenden Erfindung wird daher auch die Verwendung von Cyclodextrinen zur Stabilisierung kosmetisch oder dermatologisch unbedenklicher Substanzen, deren chemische Formel das Strukturmotiv des Dibenzoylmethans

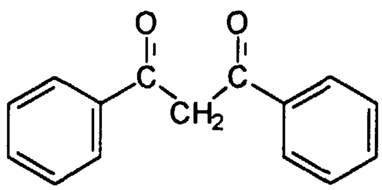

und/oder der Zimtsäure

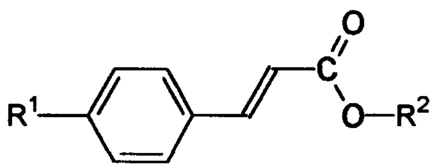

enthalten, gegen die durch UV-Strahlung induzierte Zersetzung, angesehen.

Gleichermaßen wird als bevorzugte Verkörperung der vorliegenden Erfindung auch die Verwendung von Cyclodextrinen zur Stabilisierung kosmetisch oder dermatologisch unbedenklicher Substanzen, deren chemische Formel das Strukturmotiv des Dibenzoylmethans

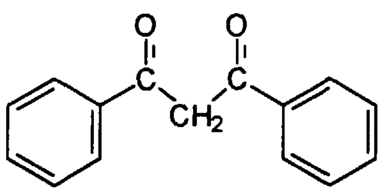

und/oder der Zimtsäure

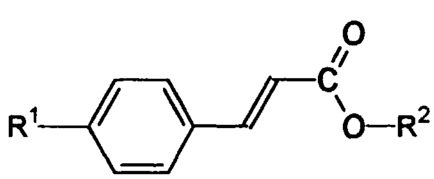

enthalten enthalten, gegen die durch UV-Strahlung induzierte Zersetzung, angesehen.Die Gesamtmenge an Dibenzoylmethanen, insbesondere 4-(tert.-Butyl)-4'-methoxydibenzoylmethan in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Zimtsäurederivaten, insbesondere 4-Methoxyzimtsäure-2'-ethylhexylester in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Cyclodextrinen, insbesondere β-Cyclodextrin und/oder γ-Cyclodextrin in den fertigen kosmetischen oder dermatologischen Zubereitungen wird vorteilhaft aus dem Bereich von 0,1 - 20,0 Gew.-%, bevorzugt 0,5 - 12,0 Gew.-% gewählt, bezogen auf das Gesamtgewicht der Zubereitungen.

Besonders vorteilhaft ist es, Gewichtsverhältnisse von Cyclodextrinen zu Dibenzoylmethanen wie 8 : 1 bis 1 : 5, bevorzugt wie 4 : 1 bis 1 : 2, besonders bevorzugt wie 3 : 1 bis 1 : 1 zu wählen.

Besonders vorteilhaft ist es ferner, Gewichtsverhältnisse von Cyclodextrinen zu Zimtsäurederivaten wie 8 : 1 bis 1 : 5, bevorzugt wie 4 : 1 bis 1 : 2, besonders bevorzugt wie 3 : 1 bis 1 : 1 zu wählen.

Insbesondere vorteilhaft sind als molekulare Addukte aus Cyclodextrinen und Dibenzoylmethanderivaten bzw. molekulare Addukte aus Cyclodextrinen und Zimtsäurederivaten angesehene Wirkstoffkombinationen welche die folgenden molaren Verhältnisse aufweisen:

1 mol β-Cyclodextrin : 1 mol Dibenzoylmethanderivat
1 mol γ-Cyclodextrin : 1 mol Dibenzoylmethanderivat
1 mol β-Cyclodextrin : 1 mol Zimtsäurederivat
1 mol γ-Cyclodextrin : 2 mol Zimtsäurederivat

Vorteilhaft ist es gewünschtenfalls, Gewichtsverhältnisse von Dibenzoylmethanen zu Zimtsäurederivaten wie 5 : 1 bis 1 : 10, bevorzugt wie 3 : 1 bis 1 : 3, zu wählen.

Erfindungsgemäße kosmetische und dermatologische Zubereitungen enthalten außerdem vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Die anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m(RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl,

Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Es kann auch von Vorteil sein, UVA-Filter einzusetzen, die bisher üblicherweise in kosmetischen Zubereitungen enthalten sind. Es können die für die UVB-Kombination verwendeten Mengen eingesetzt werden.

Ferner kann gegebenenfalls von Vorteil sein, die erfindungsgemäßen Wirkstoffkombinationen mit weiteren UVA- und/oder UVB-Filtern zu kombinieren, beispielsweise bestimmten Salicylsäurederivaten wie

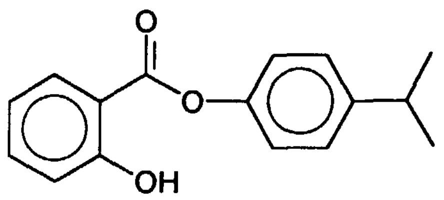

(4-Isopropylbenzylsalicylat),

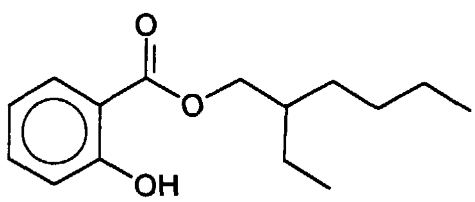

(2-Ethylhexylsalicylat, Octylsalicylat),

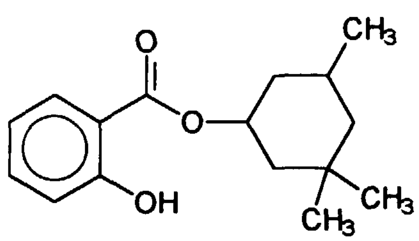

(Homomenthylsalicylat).

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

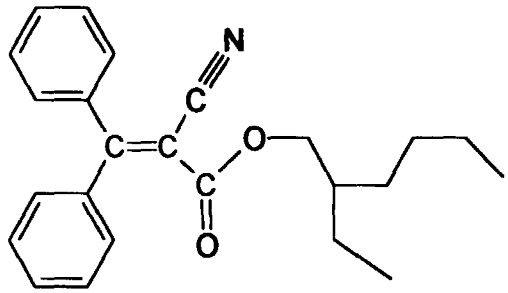

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgeweht der Zubereitungen bezogen.

**Herstellungsbeispiel 1**

In eine gesättigte wäßrige β-Cyclodextrin-Lösung wird unter intensivem Rühren bei Raumtemperatur ein Moläquivalent, bezogen auf die Cyclodextrinmenge, 4-(tert.-Butyl)-4'-methoxydibenzoyl-methan langsam zugegeben. Es wird über Nacht weiter gerührt. Das ausgefallene Produkt (= molekulares Addukt 1) wird über Keramikfilter unter Vakuum filtriert, anschließend getrocknet und zu mikronisierten Partikeln gemahlen.

**Herstellungsbeispiel 2**

In eine gesättigte wäßrige β-Cyclodextrin-Lösung wird unter intensivem Rühren bei Raumtemperatur ein Moläquivalent, bezogen auf die Cyclodextrinmenge, 4-Methoxyzimtsäure-2-ethyl-hexylester langsam zugegeben. Es wird über Nacht weiter gerührt. Das ausgefallene Produkt (= molekulares Addukt 2) wird über Keramikfilter unter Vakuum filtriert, anschließend getrocknet und zu mikronisierten Partikeln gemahlen.

**Herstellungsbeispiel 3**

In eine gesättigte wäßrige γ-Cyclodextrin-Lösung wird unter intensivem Rühren bei Raumtemperatur ein Moläquivalent, bezogen auf die Cyclodextrinmenge, 4-(tert.-Butyl)-4'-methoxydibenzoyl-methan langsam zugegeben. Es wird über Nacht weiter gerührt. Das ausgefallene Produkt (= molekulares Addukt 3) wird über Keramikfilter unter Vakuum filtriert, anschließend getrocknet und zu mikronisierten Partikeln gemahlen.

**Herstellungsbeispiel 4**

In eine gesättigte wäßrige γ-Cyclodextrin-Lösung wird unter intensivem Rühren bei Raumtemperatur ein Moläquivalent, bezogen auf die Cyclodextrinmenge, 4-Methoxyzimtsäure-2-ethyl-hexylester langsam zugegeben. Es wird über Nacht weiter gerührt. Das ausgefallene Produkt (= molekulares Addukt 4) wird über Keramikfilter unter Vakuum filtriert, anschließend getrocknet und zu mikronisierten Partikeln gemahlen.

**Herstellungsbeispiel 5**

In eine gesättigte wäßrige γ-Cyclodextrin-Lösung werden unter intensivem Rühren bei Raumtemperatur zwei Moläquivalente, bezogen auf die Cyclodextrinmenge, 4-Methoxyzimtsäure-2-ethyl-hexylester langsam zugegeben. Es wird über Nacht weiter gerührt. Das ausgefallene Produkt (= molekulares Addukt 5) wird über Keramikfilter unter Vakuum filtriert, anschließend getrocknet und zu mikronisierten Partikeln gemahlen.

| **Beispiel 1**<br>O/W-Lotion | | |
|---|---|---|
| | 1a | 1b |
| | Gew.-% | Gew.-% |
| Glycerylstearat | 3,50 | 3,50 |
| Stearinsäure | 1,80 | 1,80 |
| Glycerin | 3,00 | 3,00 |
| Cetylstearylalkohol | 0,50 | 0,50 |
| Natriumhydroxid (45 %ig) | 0,20 | 0,20 |
| Octyldodecanol | 7,00 | 7,00 |
| Dicaprylether | 8,00 | 8,00 |
| 2,4,6-Trianilino-(p-carbo-2'-ethyl-1'-hexyloxy)-1,3,5-triazin | 3,00 | 3,00 |
| molekulares Addukt 1 | 5,00 | - |
| molekulares Addukt 3 | - | 5,00 |
| molekulares Addukt 2 | 5.00 | - |
| molekulares Addukt 4 | - | 5.00 |
| Heptansäure-2.2-dimethyl-1.3-propandioldiester | 6,00 | 6,00 |
| Carbomer | 0,20 | 0,20 |
| Konservierungsmittel | q.s. | q.s. |
| Parfum | q.s. | q.s. |
| Wasser, demin. | ad | ad |
| | 100,00 | 100,00 |

| **Beispiel 2**<br>Hydrodispersionsgel | | |
|---|---|---|
| | 2a | 2b |
| | Gew.-% | Gew.-% |
| Pemulen TR-1 | 0,50 | 0,50 |
| Ethanol | 3,50 | 3,50 |
| Glycerin | 3,00 | 3,00 |
| Dimethicon | 1,50 | 1,50 |
| Natriumhydroxid (45 %ig) | 0,55 | 0,55 |
| Octyldodecanol | 0,50 | 0,50 |
| Capric/Caprylic Triglyceride | 5,00 | 5,00 |
| Heptansäure-2.2-dimethyl-1.3-propandiolester | 5,00 | 5,00 |

(fortgesetzt)

| **Beispiel 2**<br>Hydrodispersionsgel | | |
|---|---|---|
| | 2a | 2b |
| | Gew.-% | Gew.-% |
| 4-Methyl-Benzylidencampher | 4,00 | 4,00 |
| Octocrylen | 10,00 | 10,00 |
| 4-Methoxyzimtsäure-2-ethyl-hexylester | 5,00 | 5,00 |
| molekulares Addukt 1 | 8.00 | - |
| molekulares Addukt 3 | - | 8.00 |
| Carbomer | 0,20 | 0,20 |
| Konservierungsmittel | q.s. | q.s. |
| Parfum | q.s. | q.s. |
| Wasser, demin. | ad | ad |
| | 100,00 | 100,00 |

| **Beispiel 3**<br>O/W-Creme | | |
|---|---|---|
| | 3a | 3b |
| | Gew.-% | Gew.-% |
| Glycerylstearat SE | 3,50 | 3,50 |
| Stearinsäure | 3,50 | 3,50 |
| Butylenglykol | 5,00 | 5,00 |
| Cetylstearylalkohol | 3,00 | 3,00 |
| Natriumhydroxid (45 %ig) | 0,35 | 0,35 |
| $C_{12}$-$C_{15}$ Alkylbenzoate | 10,00 | 10,00 |
| 2,4,6-Trianilino-(p-carbo-2'-ethyl-1'-hexloxy)-1,3,5-triazin | 4,00 | 4,00 |
| molekulares Addukt 1 | 8.00 | - |
| molekulares Addukt 3 | - | 8.00 |
| $TiO_2$ | 3,00 | 3,00 |
| Octyldodecanol | 6,00 | 6,00 |
| Carbomer | 0,20 | 0,20 |
| Konservierungsmittel | q.s. | q.s. |
| Parfum | q.s. | q.s. |
| Wasser, demin. | ad | ad |
| | 100,00 | 100,00 |

| **Beispiel 4** W/O-Lotion | | |
|---|---|---|
| | 4a | 4b |
| | Gew.-% | Gew.-% |
| Polyglceryl-2-Polyhydroxystearat | 3,50 | 3,50 |
| Polyglyceryl-3-Diisostearat | 3,50 | 3,50 |
| Butylenglykol | 5,00 | 5,00 |
| Ceresin | 3,00 | 3,00 |
| Natriumhydroxid (45 %ig) | 0,35 | 0,35 |
| $C_{12}$-$C_{15}$ Alkylbenzoate | 10,00 | 10,00 |
| Octocrylen | 10,00 | 10,00 |
| molekulares Addukt 2 | 10,00 | - |
| molekulares Addukt 4 | | 10,00 |
| molekulares Addukt 1 | 5,00 | - |
| molekulares Addukt 3 | | 5,00 |
| Phenylbenzimidazol-5-sulfonsäure | 2,00 | 2,00 |
| NaOH | q.s. | q.s. |
| Miglyol 812 | 6,00 | 6,00 |
| Vaseline | 2,00 | 2,00 |
| Konservierungsmittel | q.s. | q.s. |
| Parfum | q.s. | q.s. |
| Wasser, demin. | ad | ad |
| | 100,00 | 100,00 |

| **Beispiel 5** O/W-Lotion | | |
|---|---|---|
| | 5a | 5b |
| | Gew.-% | Gew.-% |
| Glycerylstearat | 3,50 | 3,50 |
| Stearinsäure | 1,80 | 1,80 |
| Glycerin | 3,00 | 3,00 |
| Cetylstearylalkohol | 0,50 | 0,50 |
| Natriumhydroxid (45 %ig) | 0,20 | 0,20 |
| Octyldodecanol | 7,00 | 7,00 |
| Dicaprylylether | 8,00 | 8,00 |
| 2,4,6-Trianilino-(p-carbo-2'-ethyl-1'-hexyloxy)-1,3,5-triazin | 4,00 | 4,00 |

(fortgesetzt)

| **Beispiel 5** O/W-Lotion | | |
|---|---|---|
| | 5a | 5b |
| | Gew.-% | Gew.-% |
| $TiO_2$(hydrophob) | 4,00 | 4,00 |
| molekulares Addukt 1 | 5,00 | - |
| molekulares Addukt 3 | - | 5,00 |
| 4-Methyl-Benzylidencampher | 2,00 | 2,00 |
| Benzophenon 3 | 4,00 | 4,00 |
| Tricaprylin | 6,00 | 6,00 |
| Carbomer | 0,20 | 0,20 |
| Konservierungsmittel | q.s. | q.s. |
| Parfum | q.s. | q.s. |
| Wasser, demin. | ad | ad |
| | 100,00 | 100,00 |

| **Beispiel 6** Hydrodispersionsgel | | |
|---|---|---|
| | 6a | 6b |
| | Gew.-% | Gew.-% |
| Pemulen TR-1 | 0,50 | 0,50 |
| Ethanol | 3,50 | 3,50 |
| Glycerin | 3,00 | 3,00 |
| Dimethicon | 1,50 | 1,50 |
| Natriumhydroxid (45 %ig) | 0,55 | 0,55 |
| Octyldodecanol | 0,50 | 0,50 |
| Capric/Caprylic Triglyceride | 5,00 | 5,00 |
| Arlasolv DMI | 5,00 | 5,00 |
| 2,4,6-Trianilino-(p-carbo-2'-ethyl-1'-hexyloxy)-1,3,5-triazin | 5,00 | 5,00 |
| $TiO_2$(hydrophob) | 2,00 | 2,00 |
| molekulares Addukt 1 | 5,00 | - |
| molekulares Addukt 3 | - | 5,00 |
| Carbomer | 0,20 | 0,20 |
| Konservierungsmittel | q.s. | q.s. |
| Parfum | q.s. | q.s. |
| Wasser, demin. | ad | ad |
| | 100,00 | 100,00 |

| **Beispiel 7**<br>O/W-Creme | | |
|---|---|---|
| | 7a | 7b |
| | Gew.-% | Gew.-% |
| Glycerylstearat SE | 3,50 | 3,50 |
| Stearinsäure | 3,50 | 3,50 |
| Butylenglykol | 5,00 | 5,00 |
| Cetylstearylalkohol | 3,00 | 3,00 |
| Natriumhydroxid (45 %ig) | 0,35 | 0,35 |
| $C_{12}$-$C_{15}$ Alkylbenzoate | 10,00 | 10,00 |
| 2,4,6-Trianilino-(p-carbo-2'-ethyl-1'-hexyloxy)-1,3,5-triazin | 4,00 | 4,00 |
| $TiO_2$(hydrophob) | 4,00 | 4,00 |
| molekulares Addukt 1 | 10.00 | - |
| molekulares Addukt 3 | - | 10.00 |
| Cosmacol ELI | 6,00 | 6,00 |
| Carbomer | 0,20 | 0,20 |
| Konservierungsmittel | q.s. | q.s. |
| Parfum | q.s. | q.s. |
| Wasser, demin. | ad | ad |
| | 100,00 | 100,00 |

| **Beispiel 8**<br>W/O-Lotion | | |
|---|---|---|
| | 8a | 8b |
| | Gew.-% | Gew.-% |
| Polyglyceryl-2-Polyhydroxystearat | 3,50 | 3,50 |
| Polyglyceryl-3-Diisostearat | 3,50 | 3,50 |
| Butylenglykol | 5,00 | 5,00 |
| Ceresin | 3,00 | 3,00 |
| Natriumhydroxid (45 %ig) | 0,35 | 0,35 |
| $C_{12}$-$C_{15}$ Alkylbenzoate | 10,00 | 10,00 |
| 2,4,6-Trianilino-(p-carbo-2'-ethyl-1'-hexyloxy)-1,3,5-triazin | 4,00 | 4,00 |
| $TiO_2$(hydrophob) | 2,00 | 2,00 |
| molekulares Addukt 1 | 5,00 | - |
| molekulares Addukt 3 | - | 5,00 |

(fortgesetzt)

| Beispiel 8 W/O-Lotion | | |
|---|---|---|
| | 8a | 8b |
| | Gew.-% | Gew.-% |
| molekulares Addukt 2 | 8,00 | - |
| molekulares Addukt 4 | - | 8,00 |
| Cosmacol ELI | 6,00 | 6,00 |
| Vaseline | 2,00 | 2,00 |
| Konservierungsmittel | q.s. | q.s. |
| Parfum | q.s. | q.s. |
| Wasser, demin. | ad | ad |
| | 100,00 | 100,00 |

| Beispiel 9 W/O-Lotion | | |
|---|---|---|
| | 9a | 9b |
| | Gew.-% | Gew.-% |
| Polyglyceryl-2-Polyhydroxystearat | 3,50 | 3,50 |
| Polyglyceryl-3-Diisostearat | 3,50 | 3,50 |
| Butylenglykol | 5,00 | 5,00 |
| Ceresin | 3,00 | 3,00 |
| Natriumhydroxid (45 %ig) | 0,35 | 0,35 |
| $C_{12}$-$C_{15}$ Alkylbenzoate | 10,00 | 10,00 |
| 2,4,6-Trianilino-(p-carbo-2'-ethyl-1'-hexyloxy)-1,3,5-triazin | 4,00 | 4,00 |
| $TiO_2$(hydrophob) | 2,00 | 2,00 |
| molekulares Addukt 1 | 5,00 | - |
| molekulares Addukt 3 | - | 5,00 |
| molekulares Addukt 5 | 8,00 | 8,00 |
| Cosmacol ELI | 6,00 | 6,00 |
| Vaseline | 2,00 | 2,00 |
| Konservierungsmittel | q.s. | q.s. |
| Parfum | q.s. | q.s. |
| Wasser, demin. | ad | ad |
| | 100,00 | 100,00 |

## Patentansprüche

1. Wirkstoffkombinationen aus Cyclodextrinen und kosmetisch oder dermatologisch unbedenklichen Substanzen, deren chemische Formel das Strukturmotiv des Dibenzoylmethans

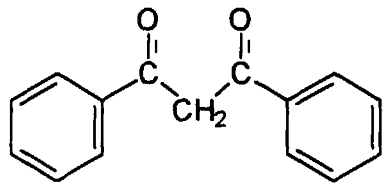

und/oder der Zimtsäure

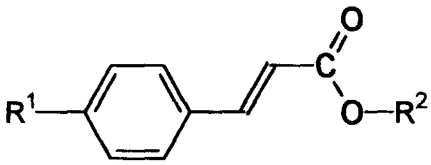

enthalten.

2. Wirkstoffkombinationen nach Anspruch 1, dadurch gekennzeichnet, daß die Cyclodextrine gewählt werden aus der Gruppe β-Cyclodextrin, γ-Cyclodextrin.

3. Wirkstoffkombinationen nach Anspruch 1, dadurch gekennzeichnet, daß die Substanzen, die das Strukturmotiv des Dibenzoylmethans aufweisen, gewählt werden aus der Gruppe 5-Isopropyldibenzoylmethan, 4-(tert.-Butyl)-4'-methoxydibenzoylmethan.

4. Wirkstoffkombinationen nach Anspruch 1, dadurch gekennzeichnet, daß die Substanzen, die das Strukturmotiv der Zimtsäure aufweisen, gewählt werden aus der Gruppe 4-Methoxyzimtsäureisopentyleste, 4-Methoxyzimtsäure-2'-ethylhexylester.

5. Wirkstoffkombinationen nach Anspruch 1, dadurch gekennzeichnet, daß sie in Form molekularer Addukte aus Cyclodextrinen und Dibenzoylmethanderivaten oder in Form molekularer Addukte aus Cyclodextrinen und Zimtsäurederivaten vorliegen.

6. Wirkstoffkombinationen in Form molekularer Addukte aus Cyclodextrinen und Dibenzoylmethanderivaten oder in Form molekularer Addukte aus Cyclodextrinen und Zimtsäurederivaten vorliegen nach Anspruch, 5 dadurch gekennzeichnet, daß sie die folgenden molaren Verhältnisse aufweisen:

   1 mol β-Cyclodextrin : 1 mol Dibenzoylmethanderivat
   1 mol γ-Cyclodextrin : 1 mol Dibenzoylmethanderivat
   1 mol β-Cyclodextrin : 1 mol Zimtsäurederivat
   1 mol γ-Cyclodextrin : 2 mol Zimtsäurederivat.

7. Wirkstoffkombinationen nach Anspruch 1, insbesondere molekulare Addukte nach Anspruch 5 oder 6, dadurch gekennzeichnet, daß die Gesamtmenge an Substanzen, die das Strukturmotiv des Dibenzoylmethans aufweisen, insbesondere 4-(tert.-Butyl)-4'-methoxydibenzoylmethan, in den fertigen kosmetischen oder dermatologischen Zubereitungen aus dem Bereich von 0,1 - 10,0 Gew.-%, bevorzugt 0,5 - 6,0 Gew.-%, bezogen auf das Gesamtgewicht der Zubereitungen gewählt wird.

8. Wirkstoffkombinationen nach Anspruch 1, insbesondere molekulare Addukte nach Anspruch 5 oder 6, dadurch gekennzeichnet, daß die Gesamtmenge an Substanzen, die das Strukturmotiv der Zimtsäure aufweisen, insbesondere 4-Methoxyzimtsäure-2'-ethylhexylester, in den fertigen kosmetischen oder dermatologischen Zubereitungen aus dem Bereich von 0,1 - 10,0 Gew.-%, bevorzugt 0,5 - 6,0 Gew.-%, bezogen auf das Gesamtgewicht der Zubereitungen gewählt wird.